# EUROPEAN PATENT SPECIFICATION

(11) **EP 1 238 652 B1**
(45) Date of publication and mention of the grant of the patent: **18.11.2009**
(21) Application number: 00979022.1
(22) Date of filing: 29.11.2000
(51) Int. Cl.: A61Q 19/00, A61K 8/365

(54) **Cosmetic method for peeling of the skin**
Kosmetisches Verfahren zum Peelen der Haut
Procédé cosmetique d'exfoliation de la peau

(30) Priority: 14.12.1999 JP 35403199
(43) Date of publication of application: 11.09.2002
(73) Proprietor: SBI ALApromo CO., LTD., Tokyo (JP)
(72) Inventor: ITOH, Yoshiyasu, Tokyo 153-0063 (JP); TANAKA, Tohru, Cosmo Research Institute, Minato-ku, Tokyo 108-0023 (JP)
(74) Representative: HOFFMANN EITLE
(86) International application number: PCT/JP2000/008436
(87) International publication number: WO 2001/043716

(56) References cited:
- EP-A2- 0 714 600
- WO-A-95/05813
- WO-A-95/07077
- WO-A-96/06602
- WO-A-96/28412
- WO-A-98/30242
- WO-A1-95/24888
- JP-A- 7 053 487
- JP-A- 8 337 515
- JP-A- 10 194 925
- JP-A- 11 116 446
- US-A- 5 520 905
- STENDER ET AL.: "Photodynamic therapy with 5-aminolevulinic acid in the treatment of actinic cheilitis" BRITISH JOURNAL OF DERMATOLOGY, vol. 135, 1996, pages 454-456,

## Description

### TECHNICAL FIELD

The present invention relates to a composition for peeling. More particularly, it relates to a composition for carrying out peeling by applying a chemical to the skin followed by irradiation with light.

### BACKGROUND ART

Peeling is a method for promoting the regeneration of new skin by peeling off old skin. It has been widely used in the field of cosmetic surgery in order to aesthetically improve the skin which is in bad conditions but do not necessarily require a medical treatment. Peeling is roughly classified into chemical peeling and laser peeling.

In the chemical peeling, chemicals such as phenol, glycolic acid and trichloroacetic acid are applied to the skin, and the skin is peeled off. It is further classified into superficial peeling in which the skin is peeled off superficially and deep peeling in which the skin is peeled off deeply. Since the operation is included in chemical burn, inflammation arises after the completion of the operation. When the operation is carried out only on the skin surface layer to minimize the inflammation, only insufficient effects can be achieved. On the other hand, a strong operation frequently induces long-lasting pigmentation and rubefaction or even scar in some cases. The deep peeling is a highly invasive treatment which should be carried out under anesthesia and there arises postoperative pain after the operation.

The laser peeling is a therapeutic method wherein the skin surface is burnt by irradiating with laser beams instead of the application of chemicals. The operation should be carried out under anesthesia. Similar to the chemical peeling, the laser peeling is accompanied by inflammation. Since the operation frequently induces long-lasting pigmentation, rubefaction and scar formation particularly in case of Orientals, it should be extremely carefully applied to Orientals at the present stage.

As described above, the existing peeling methods may be associated with complications. When they are carried out while avoiding the complications, only insufficient effects can be obtained. Therefore, it has been required to establish an effective peeling method which imposes little burden on patients.
WO 98/30242 discloses a composition for photodynamic therapy which comprises a protoporphyrin precursor photochemotherapeutic agent and vascular stromalocalizing photosensitizer. The protoporphyrin precursor photochemotherapeutic agent may be 5-aminolevulinic acid. The composition may further include a surface-penetration assisting agent.
WO 96/28412 discloses the use of esters of 5-aminolevulinic acid as photosensitizing agents in photochemotherapy of disorders or abnormalities of external or internal surfaces of the body.
WO 95/07077 teaches a pharmaceutical composition for the treatment of disorders or abnormalities of external or internal surfaces of the body which comprises 5-aminolevulinic acid together with at least one surface-penetration assisting agent.
Stender et al. (British Journal of Dermatology, 1996, 135(3), pages 454-456) disclose the treatment of actinic cheilitis by photodynamic therapy with 5-aminolevulinic acid. 5-aminovulinic acid is applied topically to a patient's lip afflicted with actinic cheilitis and the lip is then irradiated with incoherent visible light. Superficial peeling is induced following irradiation.

### DISCLOSURE OF THE INTENTION

An object of the present invention is to provide an effective peeling method which imposes little burden on patients.

Accordingly, the present invention provides a non-therapeutic method for improving the skin of a human or animal by photodynamic peeling of the skin comprising
(i) applying a composition comprising at least one compound selected from 5-aminolevulinic acid and a salt thereof, a 5-aminolevulinic acid ester and a salt thereof, an N-acyl-5-aminolevulinic acid and a salt thereof, and an N-acyl-5-aminolevulinic acid ester and a salt thereof to the skin of a human or animal; and
(ii) irradiating the skin with light at a dose level of 3-10 J/cm² for 1-50 minutes to cause peeling of the skin.

Preferably the composition further comprises a percutaneous absorption accelerator.

### BEST MODE FOR CARRYING OUT THE INVENTION

It is known that 5-aminolevulinic acid (hereinafter sometimes referred to as "5-ALA"), derivatives thereof and salts thereof used in the composition for peeling according to the present invention are useful as plant growth promoters, herbicides, insecticides, sensitizers in photodynamic therapy for cancer and the like (Japanese Patent No. 2613136, Japanese Patent No. 2896963, JP-W-61-502814, JP-A-2-138201). However, it has not been known that 5-ALAs are applicable to peeling.

The 5-ALA derivatives include 5-aminolevulinic acid esters, *N*-acyl-5-aminolevulinic acids, and *N*-acyl-5-aminolevulinic acid esters (hereinafter referred to as "5-ALA esters", "*N*-acyl-5-ALAs", and "*N*-acyl-5-ALA esters", respectively).

Examples of the 5-ALA esters include esters of 5-ALA with an alkyl group which may be substituted and has a linear, branched or cyclic structure and 1 to 24 carbon atoms. Examples of the substituent of the substituted alkyl group include a hydroxyl group, an alkoxy group, a phenyl group and the like. Preferred examples of the alkyl group which may be substituted include a methyl group, an ethyl group, an n-propyl group, an isopropyl group, an n-pentyl group, an n-hexyl group, a cyclohexyl group, an n-heptyl group, an n-octyl group, an n-nonyl group, an n-dodecyl group, an n-hexadecyl gorup, a benzyl group, a phenethyl group, a 3-phenylpropyl group, a hydroxyethyl gorup, an ethoxyethyl group and the like.

The *N*-acyl-5-ALAs include compounds wherein the amino group of 5-ALA has been acylated by an acyl group having 1 to 24 carbon atoms, such as an alkanoyl group, an aromatic acyl group, a benzyloxycarbonyl group and the like. Preferred examples of the acyl group include an acetyl group, an n-propanoyl group, an n-butanoyl group, an n-pentanoyl group, an n-hexanoyl group, an n-nonanoyl group, a benzyloxycarbonyl group and the like.

Examples of the *N*-acyl-5-ALA esters include compounds having the same ester and acyl group as described above. Preferable examples include combinations of a methyl ester group and a formyl group, a methyl ester group and an acetyl group, a methyl ester group and an n-propanoyl group, a methyl ester group and an n-butanoyl group, an ethyl ester group and a formyl group, an ethyl ester group and an acetyl group, an ethyl ester group and an n-propanoyl group, an ethyl ester group and an n-butanoyl group and the like.

Examples of the salts of 5-ALA or derivatives thereof include acid addition salts, such as hydrochloride, hydrobromide, hydroiodide, phosphate, nitrate, sulfate, acetate, propionate, toluenesulfonate, succinate, oxalate, lactate, tartarate, glycolate, methanesulfonate, butyrate, valerate, citrate, fumarate, maleate, malate and the like; metal salts, such as sodium salt, potassium salt, calcium salt and the like; ammonium salts; alkylammonium salts; and the like. These salts may be used as an aqueous solution or a powder and have the same effect as 5-ALA.

The 5-ALA, derivatives thereof or salts thereof as described above may form a hydrate or a solvate. They may be used alone or in combination of two or more.

The 5-ALA or its salts can be obtained by any method of chemical synthesis, microbial production and enzymatic production (for example, see WO98/54297, USP 5,380,935). The 5-ALA derivatives or salts thereof can be produced by a publicly known chemical synthesis method described in, for example, JP-A-4-9360. Products obtained by microbial or enzymatic methods and crude products obtained by chemical synthesis may be used as such without separating or purifying any more, so long as they are free from any contaminant harmful to humans or animals.

The composition for peeling for use in the present invention may contain an additional component, so long as it contains the 5-ALA, derivatives thereof or salts thereof. The dosage form is not particularly limited, so long as it is an external preparation for skin. Examples include a dust, a solution, an ointment (including an oily ointment, an emulsified ointment, an water-soluble ointment and hydrogel), a dermatologic paste and the like. Examples of the additional component include a percutaneous absorption accelerator such as isopropyl myristate, a surfactant, alcohol, polyhydric alcohol and the like.

When an aqueous solution or an ointment is prepared, it is necessary to prepare it so as not to become alkaline in order to prevent 5-ALA, derivatives thereof or salts thereof from decomposition. The aqueous solution and the ointment are preferably prepared by adjusting pH 8 or lower, more preferably pH 7 or lower, and particularly preferably pH 6.1 or lower. When they become alkaline, decomposition can be prevented by eliminating oxygen. Taking this point into consideration, they can be used together with a base component generally used for the solution and the ointment.

In the non-therapeutic method of the present invention, the composition is applied to the target skin, and the skin is irradiated with light (i.e. photodynamic peeling).The term "photodynamic peeling" as used in the present invention means a peeling method in which a chemical is applied to the skin, followed by irradiation with light. The method for applying the composition to the skin is not particularly limited, so long as the active ingredient can be absorbed via the skin. Examples include spraying, coating, packing, iontophoresis and the like. The dose to the skin of 5-ALA, derivatives thereof or salts thereof which are the active ingredient is generally from 10 mg to 10g, preferably from 100 mg to 5 g and more preferably from 1 g to 5 g in terms of 5-ALA hydrochloride per 100 cm² of the skin. From the viewpoint of efficiency, after the application of the composition till the light irradiation, the applied skin is preferably shaded.

The skin which is applied with the composition is not particularly limited. But, the composition is applied to the skin which is to be improved from an esthetic viewpoint, for example, skin having old horny substances or epiderm, skin with spots, freckles or acne, skin suffering from deterioration in tension or gloss, wrinkled skin and skin after suffering from comedo.

Next, the skin is irradiated with light. From the viewpoint of the percutaneous absorption and metabolism of the active ingredient, it is preferable to irradiate the skin with light for 2 to 48 hours, preferably for 4 to 24 hours, after the application of the composition according to the present invention. It is also preferable to perform the light-irradiation after removing off the composition remaining on the skin.

As the light for the irradiation, it is preferable to employ visible beams around 635 nm or laser beams of about 635 nm. The irradiation dose level is from 3 to 10 J/cm². The irradiation time is from 1 to 50 minutes, preferably from 10 to 30 minutes.

After the treatment in accordance with the present invention, the treated part is rubefied and swells up for about 3 days. Subsequently, the skin surface peels off spontaneously. As a result, the peeling effects (for example, fall-off of old horny substances or epiderm, removal of spots, freckles or acne, restoration of skin tension or gloss, relief of wrinkles, amelioration of comedo) can be achieved within 3 or 4 days to 3 weeks, though the effects are different among individuals.

Although sufficient effects can be obtained by carrying out the treatment in accordance with the present invention once, it may be repeated plural times so as to further enhance the effects.

Although the mechanism of the photodynamic peeling according to the present invention still remains unknown, it is estimated as proceeding as follows. Death of cells and tissues is classified into two types, i.e., necrosis and apoptosis. The occurrence of necrosis, in which cell injury and death are caused by external factors such as chemicals or heat, induces inflammatory reactions such as vasodilation and migration of inflammatory cells toward the affected part. Subsequently, melanogenesis is accelerated in pigment cells. On the other hand, apoptosis means another form of cell death in which cells, so to speak, "suicide" because of intracellular factors. It is known that the cell death of this type is accompanied by no or little inflammation.

Chemical peeling and laser peeling are techniques based on the necrosis mechanism whereby epidermal cells are killed by chemicals or heat. Therefore, these treatments frequently induce troubles such as inflammation, pigmentation or scar formation which prolong over 3 month, 6 month or even 1 year or longer in some cases.

In contrast thereto, it is considered that epidermal cells are killed based on the apoptosis-like mechanism in photodynamic peeling. After externally administered, 5-ALA is absorbed in epidermal cells and then enters into the heme biosynthesis pathway in these cells. Next, it is converted into photosensitive substances such as protoporphyrin IX and accumulated in the cells. When exposed to exciting light, these photosensitive substances such as protoporphyrin IX form active oxygen and, in its turn, the cells "suicide". When 5-ALA salts or derivatives thereof are administered, 5-ALA would be induced therefrom in the body and the induced 5-ALA would trigger the same mechanism.

As described above, it is considered that the photodynamic peeling according to the present invention is based on the cell death by the apoptosis-like mechanism and, therefore, induces little troubles such as inflammation, pigmentation or rubefaction.

The present invention is explained in detail based on Examples. But they are described for only illustration, and the present invention is not limited thereto. Also, the following Examples were carried out under full informed consent on the basis of medical diagnosis.

### Example 1

5-ALA hydrochloride was added to a hydrophilic ointment in accordance with the Japanese Pharmacopoeia (manufactured by Yoshida Pharmaceutical Co., Ltd.) to give a concentration of 20% by weight and mixed thoroughly.

Under informed consent, 10 g of the obtained ointment was applied to the left half of the face of a 22 years old female. Then the treated part was covered with Saran Wrap (manufactured by Asahi Kasei Corporation, the same applies hereinafter) and further with aluminum foil for blocking off light.

After 4 hours, the treated part was cleansed and irradiated with an excimer dye laser (PDTEDL1 manufactured by Hamamatsu Photonics K.K.) at a wavelength of 635 nm at a dose level of 5 J/cm² for 30 minutes.

After the irradiation, edematous erythema was formed at the affected part. But, it gradually disappeared from the day 3. At the same time, a thin scab was formed on the skin surface. The scab, i.e., old epidermal components, gradually peeled off in bathing and face cleansing, and peeling was completed 3 weeks after the operation.

A comparison between the left and right parts of the face indicated that skin tension, wrinkles, pigmentation, acne spots and the like had been obviously relieved. In a follow-up survey carried out 6 months after the treatment, the treated part still remained in favorable conditions.

### Example 2

Under informed consent, 5 g of an ointment prepared in the same manner as in Example 1 was applied to the right cheek (6 cm x 6 cm) of a 24 years old female. Then, the treated part was covered with Saran Wrap and further with aluminum foil for blocking off light.

After 12 hours, the treated part was cleansed and irradiated with an excimer dye laser (PDTEDL1 manufactured by Hamamatsu Photonics) at a wavelength of 635 nm at a dose level of 5 J/cm² for 15 minutes.

Although edematous erythema was formed at the affected part after the irradiation, it gradually disappeared from the day 3. At the same time, old epidermal components gradually peeled off in bathing and face cleansing, and peeling was completed on the day 6 after the operation.

A comparison with the corresponding part in the left cheek indicated that skin tension, wrinkles, pigmentation, acne spots and the like had been obviously relieved. In a follow-up survey carried out 6 months after the treatment, the treated part still remained in favorable conditions.

### INDUSTRIAL APPLICABILITY

An effective peeling treatment which imposes little burden on patients can be attained by using the composition for peeling of the present invention.

## Claims

1. A non-therapeutic method for improving the skin of a human or animal by photodynamic peeling of the skin comprising
(i) applying a composition comprising at least one compound selected from 5-aminolevulinic acid and a salt thereof, a 5-aminolevulinic acid ester and a salt thereof, an N-acyl-5-aminolevulinic acid and a salt thereof, and an N-acyl-5-aminolevulinic acid ester and a salt thereof to the skin of a human or animal; and
(ii) irradiating the skin with light at a dose level of 3-10 J/cm² for 1-50 minutes to cause peeling of the skin.

2. A method according to Claim 1, wherein the composition further comprises a percutaneous absorption accelerator.

## Patentansprüche

1. Nicht-therapeutisches Verfahren zum Verbessern der Haut eines Menschen oder Tieres mittels fotodynamischen Peelings der Haut, umfassend
(i) Auftragen einer Zusammensetzung, die mindestens eine Verbindung umfasst, die aus 5-Aminolevulinsäure und einem Salz davon, einem 5-Aminolevulinsäureester und einem Salz davon, einer N-Acyl-5-aminolevulinsäure und einem Salz davon und einem N-Acyl-5-aminolevulinsäureester und einem Salz davon ausgewählt ist, auf die Haut eines Menschen oder Tieres; und
(ii) Bestrahlen der Haut mit Licht bei einem Dosis-Niveau von 3 bis 10 J/cm² über 1 bis 50 Minuten, um ein Peeling der Haut zu bewirken.

2. Verfahren gemäß Anspruch 1, worin die Zusammensetzung weiterhin einen perkutanen Absorptionsbeschleuniger umfasst.

## Revendications

1. Procédé non thérapeutique de soin de la peau d'un être humain ou d'un animal par exfoliation photodynamique de la peau, comprenant les étapes consistant à :
(i) appliquer sur la peau d'un être humain ou d'un animal, une composition comprenant au moins un composé choisi parmi l'acide 5-aminolévulinique et un sel de celui-ci, un ester de l'acide 5-aminolévulinique et un sel de celui-ci, un acide N-acyl-5-aminolévulinique et un sel de celui-ci, et un ester d'acide N-acyl-5-aminolévulinique et un sel de celui-ci, et
(ii) irradier la peau avec une lumière à une dose de 3-10 J/cm² pendant 1-50 minutes, pour produire une exfoliation de la peau.

2. Procédé selon la revendication 1, dans lequel la composition comprend en outre un accélérateur d'absorption percutanée.
